# FASCICULE DE BREVET EUROPEEN

(11) **EP 3 006 082 B1**
(45) Date de publication et mention de la délivrance du brevet: **21.06.2017**
(21) Numéro de dépôt: 15167247.4
(22) Date de dépôt: 12.05.2015
(51) Int. Cl.: A61N 1/375, A61N 1/372, A61N 1/362, A61M 25/00

(54) **ACCESSOIRE D'EXPLANTATION DE CAPSULE INTRACORPORELLE**
ZUBEHÖR ZUR ENTFERNUNG EINER IMPLANTIERTEN INTRAKORPORALEN KAPSEL
ACCESSORY FOR EXPLANTATION OF AN INTRACORPOREAL CAPSULE

(30) Priorité: 06.10.2014 FR 1459570
(43) Date de publication de la demande: 13.04.2016
(73) Titulaire: Sorin CRM SAS, 92140 Clamart (FR)
(72) Inventeur: D'Hiver, Philippe, 92320 CHATILLON (FR); Ollivier, Jean-François, 91190 VILLIERS LE BACLE (FR); Shan, Nicolas, 91260 Juvisy sur Orge (FR)
(74) Mandataire: Grünecker Patent- und Rechtsanwälte PartG mbB

(56) Documents cités:
- WO-A1-2012/058067
- WO-A1-2012/082755
- WO-A2-2011/057210
- US-A1- 2013 103 047

## Description

L'invention concerne les "dispositifs médicaux implantables actifs" tels que définis par la directive 90/385/CEE du 20 juin 1990 du Conseil des communautés européennes, notamment les implants permettant de surveiller en continu le rythme cardiaque et délivrer si nécessaire au coeur des impulsions électriques de stimulation, de resynchronisation et/ou de défibrillation en cas de trouble du rythme détecté par le dispositif.

L'invention concerne notamment, mais de manière non limitative, ceux de ces dispositifs qui se présentent sous forme d'une capsule autonome destinée à être implantée dans une cavité cardiaque (ventricule ou oreillette, à droite ou à gauche).

Ces capsules sont dépourvues de toute liaison mécanique à un dispositif principal implanté (tel qu'un boitier de générateur d'impulsions de stimulation) ou non implanté (périphérique externe tel que programmateur ou dispositif de *monitoring* pour le suivi à distance du patient), et sont dénommées pour cette raison "capsules *leadless",* pour les distinguer des électrodes ou des capteurs disposés à l'extrémité distale d'une sonde (*lead*) conventionnelle, qui est parcourue sur toute sa longueur par un ou plusieurs conducteurs reliant par voie galvanique l'électrode ou le capteur à un générateur connecté à une extrémité opposée, proximale, de la sonde. On notera toutefois que le caractère autonome de la capsule n'est pas intrinsèquement une caractéristique nécessaire de l'invention. L'invention est applicable à l'explantation de telles capsules pourvues à leur extrémité distale d'un organe d'ancrage de type vis hélicoïdale, prolongeant axialement le corps de la capsule et destiné à pénétrer dans le tissu cardiaque par vissage au moment de l'implantation au site choisi. L'explantation est particulièrement délicate car il convient, d'une part, d'arriver à saisir le corps de la capsule au moyen d'un accessoire d'explantation et, d'autre part, d'exercer sur ce corps un couple de rotation permettant de le détacher du site d'implantation où il était maintenu par la vis d'ancrage, ce couple étant suffisamment important pour vaincre les résistances et adhérences résultant des tissus fibreux qui se sont formés localement.

On connait des accessoires d'explantation désignés "lassos" ou *snares,* qui sont couramment utilisés pour capturer et retirer du matériel médical tel que corps de sonde, cathéters défectueux, guides, etc. hors des cavités cardiaques ou du système veineux. Ces lassos sont constitués d'un fil métallique souple terminé à son extrémité distale par une boucle déformable en métal à mémoire de forme, la boucle s'étendant à l'état libre dans un plan généralement perpendiculaire au fil métallique qui la porte. Le fil métallique est introduit dans l'orifice distal d'un cathéter, traversant celui-ci jusqu'à émerger côté proximal. La traction du fil depuis cette extrémité proximale du cathéter a pour effet, à l'autre extrémité, de tirer la boucle en la faisant progressivement entrer dans le cathéter où elle va se trouver logée.

L'opération consiste à introduire le cathéter dans le corps du patient, avec la boucle entièrement repliée dans la zone d'extrémité distale. La boucle est ensuite déployée hors du cathéter en repoussant le fil depuis l'extrémité proximale : du fait de la mémoire de forme du métal, la boucle reprend alors sa forme de lasso incliné par rapport à la direction du fil et du cathéter. Ce lasso peut être orienté à volonté pour capturer l'élément à extraire. Une traction exercée sur le fil permet alors de faire rentrer partiellement la boucle dans le cathéter, ce qui a pour effet d'en réduire la dimension et d'assurer ainsi le serrage de l'élément à retirer.

Le WO 2012/082755 A1 décrit un accessoire d'explantation de capsule *leadless* basé sur ce principe, qui met en oeuvre un cathéter muni d'un mécanisme à lasso pour permettre la capture de la capsule avec, à l'extrémité distale du cathéter, un élément d'accostage pouvant se coupler à l'extrémité proximale de la capsule. Lorsque la boucle du lasso est resserrée, l'organe d'accostage du cathéter vient se coupler à la capsule, autorisant la transmission d'un couple de dévissage puis l'extraction de la capsule, qui reste solidarisée à l'organe d'accostage.

L'inconvénient de ce type de dispositif réside dans le risque qu'il y a, lors de la manipulation du lasso, de saisir des tissus valvulaires ou des filaments présents au voisinage de la capture, en particulier au moment du serrage de la boucle du lasso. Les tissus, notamment les tissus valvulaires, capturés en même temps que la capsule risquent fortement d'être endommagés au moment de l'extraction, cet endommagement des tissus étant irréversible.

Ces risques sont accrus par le manque de visibilité durant l'opération, qui est réalisée par le praticien, sous amplificateur de brillance couplé à un équipement à rayons X, à distance du site d'explantation (typiquement, avec un accès fémoral pour l'introduction du cathéter destiné à atteindre la cavité ventriculaire droite), et par la difficulté qu'il y a de contrôler la fixation du lasso à la capsule avant le retrait de l'ensemble.

Le but de l'invention est de proposer un accessoire d'explantation perfectionné qui, entre autres, évite l'accrochage de filaments de tissu valvulaire au moment de la capture de la capsule par le lasso, et de l'accostage du cathéter avec la partie proximale de la capsule.

À cet effet, l'invention propose un accessoire d'explantation de capsule intracorporelle comprenant, de manière en elle-même connue d'après le WO 2012/082755 A1 précité, un corps tubulaire pourvu à son extrémité distale d'un organe d'ancrage à vis apte à pénétrer dans un tissu d'une paroi d'un organe d'un patient. L'accessoire d'explantation comprend : un cathéter, avec un tube creux portant à son extrémité distale une tête téléorientable comprenant un réceptacle tubulaire dirigé axialement et définissant un volume intérieur apte à loger la capsule au moins dans la partie proximale de celle-ci; et un lasso comprenant un fil souple s'étendant le long du cathéter et formant à son extrémité distale une boucle déformable émergeant du cathéter. La boucle du lasso s'étend entre deux extrémités configurées sur deux extrémités opposées du tube creux du cathéter, l'une au moins de ces extrémités de la boucle étant mobile par rapport au cathéter de manière à permettre un serrage de la boucle sous l'effet d'une traction exercée sur le fil souple en direction proximale le long du cathéter, et *vice versa.*

De façon caractéristique de l'invention, le bord d'extrémité libre du réceptacle tubulaire comprend : une partie antérieure proéminente ; une partie postérieure en retrait axialement par rapport à la partie antérieure et située diamétralement opposée à la partie antérieure proéminente ; et deux rebords en biseau reliant la partie antérieure proéminente à la partie postérieure en retrait.

Selon diverses caractéristiques subsidiaires avantageuses :
- la partie antérieure proéminente s'étend angulairement sur un secteur d'au moins 90° ;
- l'une des extrémités de la boucle du lasso est une extrémité fixe solidaire de l'un des rebords en biseau du réceptacle tubulaire, et l'autre extrémité de la boucle du lasso est une extrémité mobile située sur le rebord en biseau opposé ;
- les extrémités de la boucle du lasso sont toutes deux des extrémités mobiles, situées respectivement sur des rebords en biseau opposés ;
- dans ce dernier cas, la distance entre, d'une part, l'emplacement sur le rebord en biseau de l'extrémité fixe et/ou mobile de la boucle du lasso et, d'autre part, la partie postérieure en retrait, est comprise entre 20 et 40 % de la longueur du corps tubulaire de la capsule, et/ou est comprise entre 5 et 20 mm pour l'explantation d'une capsule dont la longueur du corps tubulaire est de 35 mm ;
- l'emplacement sur le rebord en biseau de l'extrémité fixe et/ou mobile de la boucle du lasso est situé à une distance comprise entre 40 et 60 % de la distance séparant les extrémités opposées de la partie antérieure proéminente et de la partie postérieure en retrait ;
- la longueur maximale de la boucle en configuration déployée est comprise entre 3 et 40 mm, pour l'explantation d'une capsule dont le diamètre du corps tubulaire est de 6,5 mm ;
- l'extrémité distale de la partie antérieure proéminente porte un marqueur radio-opaque ;
- l'accessoire comprend en outre un sous-cathéter mobile téléscopiquement dans le tube du cathéter téléorientable et pourvu à son extrémité distale de moyens de solidarisation en rotation et en translation à la capsule dans la région proximale de celle-ci.

On va maintenant décrire un exemple de mise en oeuvre de la présente invention, en référence aux dessins annexés où les mêmes références désignent d'une figure à l'autre des éléments identiques ou fonctionnellement semblables.
La Figure 1 est une vue en perspective de l'extrémité distale de l'accessoire d'explantation de l'invention avec la boucle du lasso de capture entourant la capsule à explanter, en début de manoeuvre avant serrage de cette boucle.
La Figure 2 est une vue de détail de la partie distale du réceptacle de l'accessoire d'explantation, avec la boucle du lasso de capture.
La Figure 3 est une vue frontale de la partie de réceptacle illustrée Figure 2, avec en coupe une capsule enserrée par la boucle du lasso.
La Figure 4 illustre la poignée de manoeuvre de l'accessoire d'explantation, située à l'extrémité proximale de cet accessoire et au moyen de laquelle le praticien contrôle l'ensemble de la manoeuvre.
La Figure 5 est une vue partiellement en coupe du réceptacle de l'accessoire d'explantation de l'invention avec la capsule retenue à l'intérieur par la boucle du lasso, cet ensemble étant illustré dans la phase d'approche du sous-cathéter qui permettra le dévissage de la capsule après couplage à cette dernière.
La Figure 6 est une vue en élévation de la capsule, considérée isolément, montrant le corps tubulaire muni d'une gorge de retenue de la boucle du lasso.
La Figure 7 illustre différents profils de gorge de retenue possibles pour la capsule illustrée Figure 6.
La Figure 8 est une coupe en section droite du cathéter, montrant les deux canaux pour les câbles de téléorientation du cathéter et le canal pour le câble de serrage du lasso.
La Figure 9 est un organigramme détaillant les différentes étapes du mode opératoire d'explantation de la capsule au moyen de l'accessoire de l'invention.

On va maintenant décrire un exemple, non limitatif, de mise en oeuvre de l'invention.

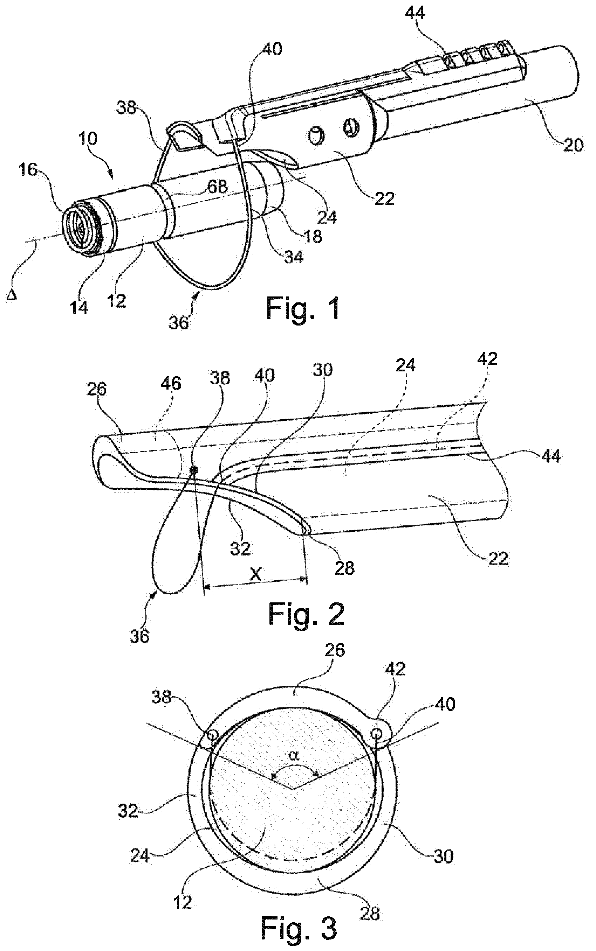

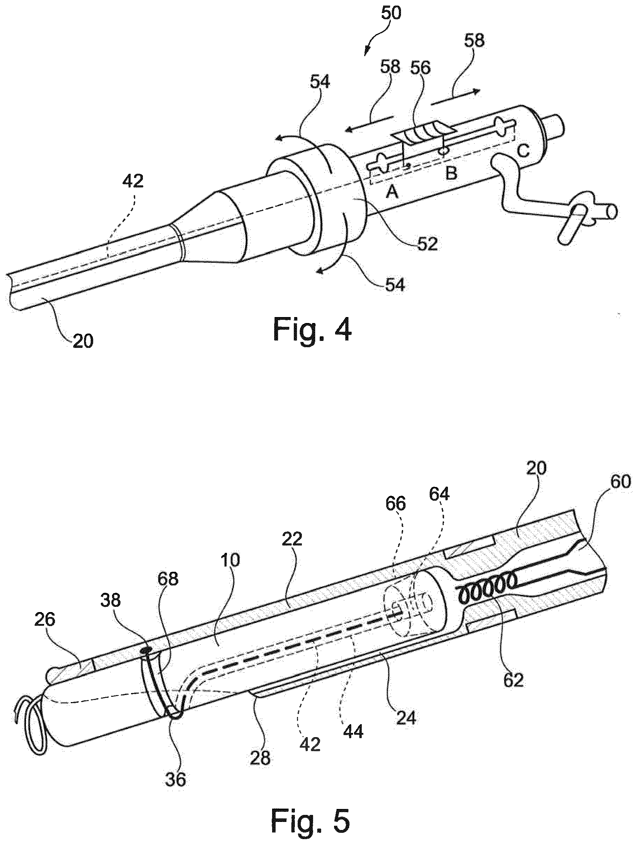

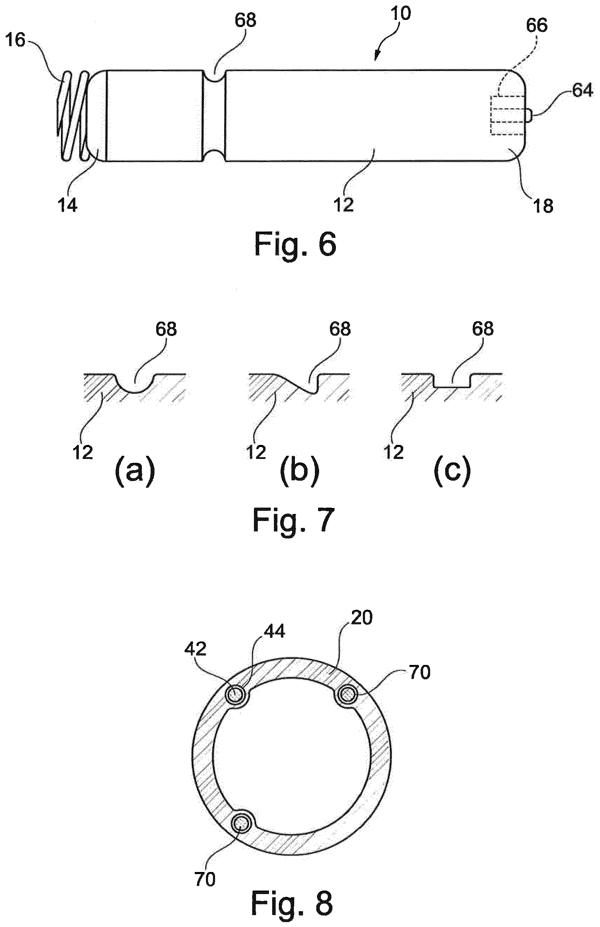

La capsule *leadless* 10, illustrée Figures 1, 5 et 6, comprend un corps tubulaire cylindrique 12 d'axe Δ, enfermant les différents circuits électroniques et d'alimentation de la capsule. Les dimensions typiques d'une telle capsule sont un diamètre de l'ordre de 6,5 mm environ pour une longueur de 35 mm environ. À son extrémité distale 14, la capsule porte une vis d'ancrage hélicoïdale 16 permettant sa fixation dans les tissus, par exemple contre une paroi d'une cavité cardiaque après vissage au moment de l'implantation. Cette vis peut éventuellement être une vis active, électriquement conductrice, pour le recueil de potentiels de dépolarisation cardiaque et/ou l'application d'impulsions de stimulation. La région proximale 18 de la capsule 10 présente de préférence une extrémité arrondie, atraumatique, et elle est pourvue d'un organe de couplage à un sous-cathéter d'implantation ou d'explantation, qui sera décrit plus bas en référence aux Figures 5 et 6.

L'accessoire d'explantation selon l'invention comprend un cathéter téléorientable (*steerable*), qui est un accessoire en lui-même connu et tout à fait conventionnel, comportant un tube de cathéter manipulé depuis son extrémité proximale par une poignée de manoeuvre (illustrée Figure 4) à la disposition du praticien, qui peut créer et régler une courbure dans la région distale du cathéter afin d'orienter l'extrémité de celui-ci en direction du site d'intervention.

De façon caractéristique de l'invention, comme illustré sur les figures, le tube de cathéter 20 est muni à son extrémité distale d'un réceptacle tubulaire 22 dirigé axialement, prolongeant le tube de cathéter et définissant un volume intérieur 24 dont la conformation et les dimensions permettent d'y loger tout ou partie de la capsule, au moins dans la région proximale 18 de celle-ci.

Le bord d'extrémité libre du réceptacle tubulaire 22 est biseauté, c'est-à-dire, plus précisément, qu'il comprend une partie antérieure proéminente 26 et, diamétralement opposée, une partie postérieure 28 axialement en retrait par rapport à la partie antérieure proéminente 26. Les deux parties antérieure 26 et postérieure 28 sont reliées par deux rebords inclinés 30, 32 réalisant la forme biseautée du bord d'extrémité du réceptacle 22. Comme illustré sur la vue frontale de la Figure 3, la partie antérieure proéminente 26 couvre angulairement un secteur formant un angle α d'au moins 90°, pour lui permettre d'assurer une fonction de guidage de la capsule au moment de l'introduction de celle-ci dans le volume intérieur 24.

Le matériau du réceptacle 22 est choisi suffisamment souple pour ne pas être traumatique à l'égard des tissus environnants lors de l'opération d'explantation. Il peut par exemple s'agir d'un élastomère thermoplastique tel que le PEBA (copolymère bloc éther-amide), d'un LDPE (polyéthylène basse densité) ou d'un polyuréthanne. De plus, le matériau du réceptacle tubulaire 22 est très avantageusement chargé, dans la région de la partie antérieure proéminente (région 46 sur la Figure 2), d'un matériau radio-opaque améliorant la visibilité de l'accessoire dans cette région critique au moment de l'approche de la capsule par le réceptacle tubulaire. Ce matériau radio-opaque peut être par exemple du BaSO₄ ou du TiO₂. L'accessoire d'explantation est en outre muni d'un système de capture à lasso comprenant un fil 34 déployable de manière à former une boucle 36 dont les extrémités 38, 40 se situent dans la région du bord d'extrémité biseauté du réceptacle tubulaire 22.

Dans le mode de réalisation illustré, l'une des extrémités 38 de la boucle 36 est une extrémité fixe, solidarisée au rebord en biseau 32 par exemple par surmoulage, collage ou soudage à un insert métallique incorporé au réceptacle tubulaire 22 à cet endroit. L'extrémité opposée 40 de la boucle 36 est, en revanche, une extrémité mobile, le fil 34 constituant la boucle se prolongeant en 42 le long du réceptacle tubulaire 22 et du cathéter jusqu'à la poignée de manoeuvre (illustrée Figure 4), le fil étant guidé dans une lumière latérale excentrée 44 du cathéter sur toute la longueur de celui-ci.

Le périmètre de la boucle peut ainsi varier typiquement, dans l'application décrite, entre 3 et 40 mm.

Le fil 34, 42 du lasso de capture est avantageusement un microcâble en alliage à mémoire de forme de type nitinol, permettant au lasso de garder une forme arrondie de boucle et une orientation perpendiculaire à l'axe du cathéter une fois la boucle déployée. Il est également possible d'utiliser un matériau tel qu'un alliage MP35N ou un acier contenant un matériau radio-opaque (par exemple un alliage platine-iridium) de manière que la boucle de lasso puisse être visualisée sous un amplificateur de brillance couplé à un équipement à rayons X.

En variante, les deux extrémités 38 et 40 de la boucle 36 peuvent être des extrémités mobiles, le fil se prolongeant alors à partir de l'extrémité 38 le long du cathéter, en y étant guidé dans une seconde lumière latérale excentrée jusqu'à l'extrémité proximale du cathéter et la poignée de manoeuvre de l'accessoire.

Les positions des extrémités 38, 40 de la boucle 36 (point fixe ou débouché de la lumière latérale excentrée 44) se situent approximativement au milieu des rebords en biseau respectifs 30, 32, c'est-à-dire à mi-distance des extrémités de la partie antérieure 26 et de la partie postérieure 28. La distance X (Figure 2) séparant, en direction axiale, ces positions des extrémités 38, 40 de la partie postérieure en retrait 28 est d'environ 20 à 40 % de la longueur du corps tubulaire 12 de la capsule, et/ou présente une longueur comprise entre 5 et 20 mm pour une capsule dont la longueur du corps tubulaire est de 35 mm.

À partir de l'état libre de la boucle, où celle-ci est entièrement déployée (configuration illustrée Figure 1), si l'on exerce depuis l'extrémité proximale du cathéter une traction sur le fil 34 dans le sens proximal, 42 l'extrémité 40 de la boucle va être progressivement tirée dans la lumière latérale 44 du cathéter, avec pour conséquence une diminution progressive du périmètre de la boucle 36 procurant l'effet de "lasso" recherché afin de capturer la capsule 10. La manoeuvre est bien entendu réversible pour permettre un relâchement ou un desserrage de la boucle et un repositionnement du lasso.

Sur la Figure 4, on a représenté un exemple de la poignée de manoeuvre 50 de l'accessoire d'explantation de l'invention, cette poignée étant située à l'extrémité proximale du tube de cathéter 20. Cette poignée 50 comprend, de manière en elle-même connue, un dispositif 52 de manoeuvre de la tête téléorientable du cathéter (flèches 54), selon un mécanisme en lui-même connu qui ne sera pas décrit en détail.

La poignée de manoeuvre est également pourvue d'un curseur 56 mobile axialement entre plusieurs positions (flèches 58), ce curseur étant relié au microcâble formant le fil de commande 42 du lasso, fil dont le déplacement en direction axiale permettra de contrôler le déploiement ou la fermeture de la boucle de capture 36 à l'extrémité distale, opposée, du tube de cathéter. Ces positions sont indexées de manière à pouvoir être aisément repérées par le praticien, avec par exemple :
- position A correspondant à un périmètre de boucle maximal, utilisé lors de la phase de recherche et de capture de la capsule,
- position B correspondant à un périmètre de boucle sensiblement égal à la circonférence de la capsule, de manière à permettre un glissement sans serrage de la boucle axialement le long du corps de la capsule, et
- position C correspondant au périmètre de boucle minimal, permettant d'assurer un serrage du corps de la capsule par cette boucle.

Comme illustré Figure 5, l'accessoire d'explantation que l'on vient de décrire permet l'introduction, dans la lumière interne du tube de cathéter 20, d'un sous-cathéter 60 terminé à son extrémité distale par un organe 62 apte à se coupler à la partie proximale de la capsule 10 après que celle-ci a été complètement introduite dans le réceptacle tubulaire 22, de manière à permettre une solidarisation en rotation et en translation avec cette dernière.

L'organe de couplage du sous-cathéter 60 à la capsule 10 peut être notamment réalisé de la manière décrite dans la demande FR 13 56020 du 24.06.2013 pour *"Capsule intracardiaque et accessoire d'implantation* in situ *par voie fémorale"*, qui décrit un mécanisme comprenant un ressort hélicoïdal (ici référencé 62) utilisé en compression radiale, c'est-à-dire pour son effet de striction ou d'étranglement (effet résultant de l'augmentation du diamètre intérieur de l'hélice), et non pour ses propriétés d'élasticité en traction/compression axiale (effet résultant de l'allongement ou du rapprochement des spires du ressort). Ce ressort hélicoïdal 62 peut s'arrimer à une tige axiale 64 formée dans la partie proximale de la capsule, à l'intérieur d'un logement 66 (Figures 5 et 6). Une fois le ressort 62 enfoncé sur la tige 64, le sous-cathéter 60 et la capsule 10 sont solidarisés en rotation, permettant ainsi un dévissage aisé de la capsule 10 depuis l'extrémité opposée, proximale, du sous-cathéter 60 par une simple rotation imprimée à celui-ci.

Avantageusement, comme illustré notamment sur les Figures 5 à 7, le corps tubulaire 12 de la capsule 10 est muni d'une gorge annulaire 68. Cette gorge 68 a pour fonction d'améliorer la retenue de la capsule 10 dans le réceptacle tubulaire 22, et donc la solidarisation de ces deux éléments en évitant tout glissement relatif. La gorge 68 est formée sur le corps tubulaire à un niveau tel que, comme illustré Figure 5, une fois que la capsule 10 a été introduite à fond dans le réceptacle tubulaire 22, la gorge 68 se situe axialement au niveau des extrémités 38, 40 de la boucle 36 du lasso.

La Figure 7 illustre différentes configurations possibles (a), (b) et (c) du profil de la gorge 68, permettant d'optimiser la retenue de la capsule par le fil de la boucle du lasso. La profondeur de la gorge 68 est typiquement comprise entre 0,15 et 0,4 mm.

La Figure 8 illustre une coupe en section droite du cathéter, montrant les deux lumières latérales excentrées de téléorientation du cathéter, avec les fils de commande 70 correspondants, et le canal 44 dans lequel coulisse le fil 42 de commande de la boucle de capture 36.

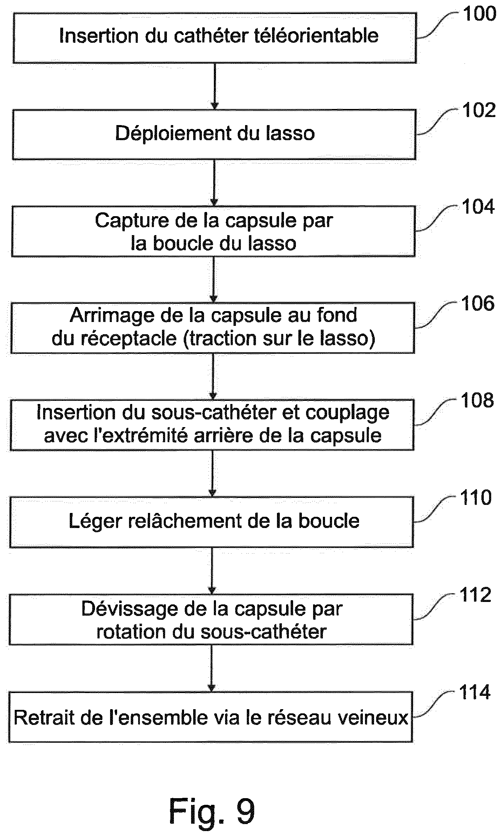

On va maintenant décrire, en référence à la Figure 9, les différentes étapes de la procédure d'explantation de la capsule au moyen de l'accessoire que l'on vient de décrire.

L'étape initiale (étape 100) consiste à insérer dans le réseau veineux le cathéter téléorientable et à guider le réceptacle tubulaire 22 jusqu'à une position proche de la capsule à extraire.

La boucle du lasso est alors déployée (étape 102) pour venir entourer le corps de la capsule.

La boucle du lasso est ensuite progressivement refermée (étape 104), de manière à rapprocher la capsule du réceptacle tubulaire 22 dans une configuration coaxiale.

La capsule est alors arrimée au fond du réceptacle 22 par une traction exercée sur la boucle du lasso (étape 106), ce qui a pour effet, compte tenu de la géométrie des différents éléments, d'exercer une traction axiale de la capsule permettant à cette dernière de s'enfoncer dans le réceptacle 22, jusqu'à venir en butée au fond de celui-ci.

Le sous-cathéter 60 peut alors être introduit (étape 108) jusqu'à ce que le ressort d'extrémité 62 vienne se coupler avec la tige 64 de la capsule. Cette dernière est fermement maintenue à l'intérieur du réceptacle tubulaire 22 pendant cette opération de couplage, du fait de la fermeture complète de la boucle 36 et de la traction exercée sur le câble 42 du lasso, qui ont pour effet d'empêcher toute sollicitation mécanique qui tendrait à repousser la capsule hors du réceptacle.

La boucle du lasso est alors légèrement relâchée (étape 110) de manière à pouvoir dévisser la capsule (étape 112), ce dévissage étant permis par le couplage en rotation et en translation du sous-cathéter 60 avec la capsule 10 au moyen du ressort 62 et de la tige 64.

L'ensemble formé par l'accessoire d'explantation et la capsule peut être alors progressivement retiré via le réseau veineux (étape 114), sans aucun risque d'arrachement ni d'endommagement des tissus environnants.

## Revendications

1. Un accessoire d'explantation de capsule intracorporelle, cette capsule (10) comprenant un corps tubulaire (12) pourvu à son extrémité distale (14) d'un organe d'ancrage à vis (16) apte à pénétrer dans un tissu d'une paroi d'un organe d'un patient,
l'accessoire d'explantation comprenant :
- un cathéter, avec un tube creux (20) portant à son extrémité distale une tête téléorientable comprenant un réceptacle tubulaire (22) dirigé axialement et définissant un volume intérieur (24) apte à loger la capsule au moins dans la partie proximale de celle-ci ; et
- un lasso, comprenant un fil souple (34, 42) s'étendant le long du cathéter et formant à son extrémité distale une boucle déformable (36) émergeant du cathéter, cette boucle s'étendant entre deux extrémités (38, 40) configurées sur deux extrémités opposées du tube creux du cathéter, l'une au moins (40) de ces extrémités de la boucle étant mobile par rapport au cathéter de manière à permettre un serrage de la boucle sous l'effet d'une traction exercée sur le fil souple en direction proximale le long du cathéter, et *vice versa*,
cet accessoire d'explantation étant **caractérisé en ce que** :
- le bord d'extrémité libre du réceptacle tubulaire comprend :
une partie antérieure proéminente (26) ;
une partie postérieure en retrait (28) axialement par rapport à la partie antérieure et située diamétralement opposée à la partie antérieure proéminente ; et
deux rebords en biseau (30, 32) reliant la partie antérieure proéminente à la partie postérieure en retrait.

2. L'accessoire d'explantation de capsule de la revendication 1, dans lequel la partie antérieure proéminente (26) s'étend angulairement sur un secteur d'au moins 90° (α).

3. L'accessoire d'explantation de capsule de la revendication 1, dans lequel l'une des extrémités de la boucle du lasso est une extrémité fixe (38) solidaire de l'un des rebords en biseau (32) du réceptacle tubulaire, et l'autre extrémité de la boucle du lasso est une extrémité mobile (40) située sur le rebord en biseau opposé (30).

4. L'accessoire d'explantation de capsule de la revendication 1, dans lequel les extrémités de la boucle du lasso sont toutes deux des extrémités mobiles, situées respectivement sur des rebords en biseau opposés.

5. L'accessoire d'explantation de capsule de la revendication 3 ou 4, dans lequel la distance (X) entre, d'une part, l'emplacement sur le rebord en biseau de l'extrémité fixe (38) et/ou mobile (40) de la boucle (36) du lasso et, d'autre part, la partie postérieure en retrait (28), est comprise entre 20 et 40 % de la longueur du corps tubulaire (12) de la capsule.

6. L'accessoire d'explantation de capsule de la revendication 3 ou 4, dans lequel la distance (X) entre, d'une part, l'emplacement sur le rebord en biseau de l'extrémité fixe (38) et/ou mobile (40) de la boucle (36) du lasso et, d'autre part, la partie postérieure en retrait (28), est comprise entre 5 et 20 mm, pour l'explantation d'une capsule dont la longueur du corps tubulaire est de 35 mm.

7. L'accessoire d'explantation de capsule de la revendication 3 ou 4, dans lequel l'emplacement sur le rebord en biseau de l'extrémité fixe (38) et/ou mobile (40) de la boucle du lasso est situé à une distance comprise entre 40 et 60 % de la distance séparant les extrémités opposées de la partie antérieure proéminente (26) et de la partie postérieure en retrait (28).

8. L'accessoire d'explantation de capsule de la revendication 1, dans lequel la longueur maximale de la boucle (36) en configuration déployée est comprise entre 3 et 40 mm, pour l'explantation d'une capsule (10) dont le diamètre du corps tubulaire est de 6,5 mm.

9. L'accessoire d'explantation de capsule de la revendication 1, dans lequel l'extrémité distale de la partie antérieure proéminente (26) porte un marqueur radio-opaque (46).

10. L'accessoire d'explantation de capsule de la revendication 1, comprenant en outre un sous-cathéter (60) mobile téléscopiquement dans le tube du cathéter téléorientable et pourvu à son extrémité distale de moyens (62) de solidarisation en rotation et en translation à la capsule dans la région proximale de celle-ci.

## Patentansprüche

1. Explantationszubehör einer intrakorporalen Kapsel, wobei diese Kapsel (10) einen schlauchförmigen Körper (12) umfasst, der an seinem distalen Ende (14) mit einem schraubenförmigen Ankerelement (16) ausgestattet ist, das in der Lage ist, in ein Gewebe einer Wand eines Organs eines Patienten einzudringen,
wobei das Explantationszubehör Folgendes umfasst:
- ein Katheter mit einem hohlen Schlauch (20), der an seinem distalen Ende einen fernausrichtbaren Kopf trägt, der einen axial geführten schlauchförmigen Behälter (22) umfasst, der ein Innenvolumen (24) bildet, das mindestens den proximalen Teil der Kapsel aufnehmen kann; und
- ein Lasso, das einen sich entlang des Katheters erstreckenden und an seinem distalen Ende eine aus dem Katheter herausragende verformbare Schlaufe (36) bildenden flexiblen Draht (34, 42) umfasst, wobei sich diese Schlaufe zwischen zwei an gegenüberliegenden Enden (38, 40) des hohlen Schlauchs des Katheters ausgebildeten Enden erstreckt, wobei mindestens eines (40) dieser Enden der Schlaufe relativ zum Katheter derart beweglich ist, dass ein Festziehen der Schlaufe durch die Ausübung einer Zugkraft auf den flexiblen Draht in der proximalen Richtung entlang des Katheters und umgekehrt ermöglicht wird,
wobei dieses Explantationszubehör **dadurch gekennzeichnet ist, dass**
- der freie Endrand des schlauchförmigen Behälters Folgendes umfasst:
• einen hervorragenden vorderen Teil (26);
• einen relativ zum vorderen Teil axial rückstehenden und dem hervorragenden vorderen Teil diametral gegenüberliegenden hinteren Teil (28); und
• zwei abgeschrägte Kanten (30, 32), die den hervorragenden vorderen Teil mit dem rückstehenden hinteren Teil verbinden.

2. Explantationszubehör einer Kapsel nach Anspruch 1, bei dem der hervorragende vordere Teil (26) sich winkelförmig über einen Bereich von mindestens 90° (α) erstreckt.

3. Explantationszubehör einer Kapsel nach Anspruch 1, bei dem eines der Enden der Schlaufe des Lassos ein mit einer der abgeschrägten Kanten (32) des schlauchförmigen Behälters fest verbundenes festes Ende (38) ist und das andere Ende der Schlaufe des Lassos ein an der gegenüberliegenden abgeschrägten Kante (30) befindliches bewegliches Ende (49) ist.

4. Explantationszubehör einer Kapsel nach Anspruch 1, bei dem beide Enden der Schlaufe des Lassos bewegliche Enden sind, die sich jeweils auf gegenüberliegenden abgeschrägten Kanten befinden.

5. Explantationszubehör einer Kapsel nach Anspruch 3 oder 4, bei dem der Abstand (X) zwischen dem Ort des festen (38) und/oder beweglichen (40) Endes der Schlaufe (36) des Lassos auf der abgeschrägten Kante einerseits und dem rückstehenden hinteren Teil (28) andererseits zwischen 20 und 40% der Länge des schlauchförmigen Körpers (12) der Kapsel beträgt.

6. Explantationszubehör einer Kapsel nach Anspruch 3 oder 4, bei dem der Abstand (X) zwischen dem Ort des festen (38) und/oder beweglichen (40) Endes der Schlaufe (36) des Lassos auf der abgeschrägten Kante einerseits und dem rückstehenden hinteren Teil (28) andererseits, zur Explantation einer Kapsel mit einem 35 mm langen schlauchförmigen Körper, zwischen 5 und 20 mm beträgt.

7. Explantationszubehör einer Kapsel nach Anspruch 3 oder 4, bei dem der Ort des festen (38) und/oder beweglichen (40) Endes der Schlaufe des Lassos auf der abgeschrägten Kante sich in einem Abstand zwischen 40 und 60% des Abstands zwischen den gegenüberliegenden Enden des hervorragenden vorderen Teils (26) und des rückstehenden hinteren Teils (28) befindet.

8. Explantationszubehör einer Kapsel nach Anspruch 1, bei dem die maximale Länge der Schlaufe (36) in ihrer ausgebreiteten Konfiguration, zur Explantation einer Kapsel (10), deren schlauchförmiger Körper einen Durchmesser von 6,5 mm aufweist, zwischen 3 und 40 mm beträgt.

9. Explantationszubehör einer Kapsel nach Anspruch 1, bei dem das distale Ende des hervorragenden vorderen Teils (26) eine strahlenundurchlässige Markierung (46) trägt.

10. Explantationszubehör einer Kapsel nach Anspruch 1, weiter umfassend ein Unter-Katheter (60), das im Schlauch des fernausrichtbaren Katheters teleskopisch beweglich ist und an seinem distalen Ende mit Mitteln (62) zur rotatorischen und translatorischen Befestigung im proximalen Bereich der Kapsel ausgestattet ist.

## Claims

1. An explantation accessory for an intracorporeal capsule, the capsule (10) comprising a tubular body (12) provided at its distal end (14) with an anchoring screw member (16) adapted to penetrate into tissue of a wall of an organ of a patient, the explantation accessory comprising:
- a catheter with a hollow tube (20) carrying at its distal end a remote steerable head comprising a tubular receptacle (22) directed axially and defining an interior volume suitable (24) for housing the capsule at least in the proximal part thereof; and
- a lasso comprising a flexible wire (34, 42) extending along the catheter and forming at its distal end a deformable loop (36) emerging from the catheter, the loop extending between two ends (38,40) configured on two opposite ends of the catheter hollow tube, at least one (40) of these ends of the loop being mobile relative to the catheter so as to allow tightening of the loop under the effect of a traction exerted on the flexible wire proximally along the catheter, and vice versa;
this explantation accessory being **characterized in that**:
- the free end edge of the tubular receptacle comprises:
• a protruding anterior portion (26);
• an posterior portion (28) axially recessed with respect to the anterior portion and located diametrically opposite to the protruding anterior portion; and
• two beveled edges (30, 32) connecting the protruding anterior portion to the recessed posterior portion.

2. The explantation accessory for a capsule of claim 1, wherein the protruding anterior portion (26) extends angularly over a sector of at least 90° (α).

3. The explantation accessory for a capsule of claim 1, wherein one end of the loop of the lasso is a fixed end (38) integral with one of the bevel edges (32) of the tubular receptacle, and the other end of the loop of the lasso is a mobile end (40) located on the opposite beveled edge (30).

4. The explantation accessory for a capsule of claim 1, wherein the ends of the lasso loop are both mobile ends, respectively located on opposite beveled edges.

5. The explantation accessory for a capsule of claim 3 or 4, wherein the distance (X) between, on the one hand, the location on the beveled edge of the fixed end (38) and/or the mobile end (40) of the loop (36) of the lasso and, on the other hand, the recessed posterior portion (28) is between 20 and 40% of the length of the tubular body (12) of the capsule.

6. The explantation accessory for a capsule of claim 3 or 4, wherein the distance (X) between, on the one hand, the location on the beveled edge of the fixed end (38) and/or the mobile end (40) of the loop (36) of the lasso and, on the other hand, the recessed posterior portion (28) is between 5 and 20 mm, for explantation of a capsule, whose tubular body length is 35 mm.

7. The explantation accessory for a capsule of claim 3 or 4, wherein the location on the beveled edge of the fixed end (38) and/or the mobile end (40) of the loop of the lasso is located at a distance between 40 and 60% of the distance between the opposite ends of the protruding anterior portion (26) and the recessed posterior portion (28).

8. The explantation accessory for a capsule of claim 1, wherein the maximum length of the loop (36) in the deployed configuration is between 3 and 40 mm, for explantation of the capsule (10), whose diameter of the tubular body is 6.5 mm.

9. The explantation accessory for a capsule of claim 1, wherein the distal end of the protruding anterior portion (26) carries a radiopaque marker (46).

10. The explantation accessory for a capsule of claim 1, further comprising a sub-catheter (60) telescopically mobile within the remote steerable catheter tube and provided at its distal end with means (62) for securing in rotation and in translation the capsule in the proximal region thereof.
